# EUROPEAN PATENT APPLICATION

(11) **EP 0 621 033 A1**
(43) Date of publication of application: **26.10.1994**
(21) Application number: 93201143.0
(22) Date of filing: 20.04.1993
(51) Int. Cl.: A61K 31/00, A61K 31/65, A61K 47/32, A61K 47/22

(54) **Antibiotic composition containing oxytetracycline and glycofurol**

(71) Applicant: Nostras S.A., 2016 Luxemburg (LU)
(72) Inventor: Megens, Gijsbertus Franciscus Josephus, B-2370 Arendonk (BE)
(74) Representative: Timmermans, Anthonius C.Th., Ir.

(57) **Abstract**

Antibiotic active aqueous composition especially for application by injection in the veterinary science containing as the active antibiotic a complex of oxytetracycline and magnesium and polyvinylpyrrolidone as stabilisator and such a percentage of ethanolamine that the pH of the composition has a value from 7.0 - 9.5 and as a solvent for the antibiotic active complex glycofurol.

## Description

The invention relates to an antibiotic active aqueous composition, especially for use by injection in the veterinary science.

Many compositions have been proposed for the above mentioned use, having widely different compositions. These compositions must fulfil many conditions, among which of course, first should be mentioned that in the treatment of living beings, especially in the treatment of animals, no adverse effects should be encountered. A further condition is that they should be easily injectable and that when injecting them, no pain and little tissue irritation should be caused. Furthermore the liquid should be stable so that it can be stocked for a long time and that no dissociations or unwanted reactions take place. Furthermore a quick and almost complete absorption at the injection point preventing the forming of a residue is important.

The European patent no. 0 096 942, describes an aqueous antibiotic preparation of the same kind which contains as the active antibiotic, a complex of Oxytetracycline and magnesium. To fulfil the above mentioned other conditions, at least to some extent, the composition according to this patent contains moreover a certain amount of polyvinylpyrrolidone and a certain amount of ethanolamine, preferably monoethanolamine, to bring the pH to a value between 6.0 and 9.5. As an antioxydant the composition may also contain sodiumformaldehydesulfoxylate.

As the antibiotic active part i.e., the oxytetracyclinemagnesiumcomplex is very poorly soluble in water, the composition according to the above mentioned patent contains a certain amount of N-methylpyrrolidone in which the complex is well soluble. Moreover the N-methylpyrolidone also brings about an improvement of the stability of the composition.

Although with the composition according to this European patent no. 0 096 942, good results have been achieved, there still exists the wish to increase the amount of the active complex as well as to improve the above indicated desirable properties of the composition. This is the aim of the invention.

An aqueous antibiotic active composition according to the invention, especially for use by injection in the veterinary science, contains as the active antibiotic a complex of oxytetracycline and magnesium as well as polyvinylpyrrolidone as a stabilisator and such a percentage of ethanolamine that the pH of the composition has a value of 7.0-9.5 and is characterized by the fact that the composition contains as solvent for the antibiotic active complex, glycofurol.

Glycofurol, also called tetrahydrofurane is a substance available on the market with the empiric formula (C₂H₄O) _{X}C₄H₈O₂, in which X is about 2. This substance is well mixable with water and soluble in ether, ethanol, propanol and glycerol and has a low toxicity.

Through the use of the glycofurol in a composition according to the invention, very little tissue damage has been observed after injection and moreover the tolerance, that is the possible quantity of injected fluid can be chosen very large. It is also observed that the injection creates hardly any painful reaction as is the case when the solvent N-methylpyrrolidone according to the above mentioned European patent is used. The N-methylpyrrolidone is in fact an agressive substance.

The composition according to the invention may contain a certain percentage of sodiumformaldehydesulfoxylate in order to increase the stability against oxidation.

To adjust the pH of the composition a certain amount of monoethanolamine can be used.

Preferably in the antibiotic active complex the ratio by weight of the oxytetracycline/magnesium lies between 2 and 15.

The composition according to the invention is especially characterized in that the following composition is fulfilled:

| | | |
|---|---|---|
| Oxytetracycline | 10-30 | % by weight |
| Magnesium | 1-15 | % by weight |
| Sodiumformaldehydesulfoxylate | 0,2-1,0 | % by weight |
| Polyvinylpyrrolidone | 2-15 | % by weight |
| Glycofurol | 10-65 | % by weight |
| Monoethanolamine to adjust to pH 7.0-9.5 | 1-8 | % by weight |
| Water for injection q.s.p. | 100 | % by weight |

By way of example an embodiment of a method for the preparation of the composition as above set out, will be given below.

### Example of preparation.

To prepare 100 l. of the composition, 45 l. glycofurol is mixed at room temperature in a suitable, for instance glass container, with about 80% of the required quantity of distilled water. Next 0.5 kg of sodiumformaldehydesulfoxylate is added and stirred. Thereafter 1.6 kg. MgO is added and is stirred with high velocity until complete solution is obtained.

Next, under slow stirring and with as less possible air inclusion 22 kg oxytetracyclinedihydrate is added. After the complete solution of all components has been achieved 5 kg polyvinylpyrrolidone is added under slow stirring and with as less air inclusion as is possible.

The mixing time will be about 5 minutes. Thereafter the pH is adjusted to 8.8 by addition of monoethanolamine. Finally distilled water is added up to 100 l. After 45 minutes the pH is again controlled and if necessary adjusted anew to 8.8.

The fluid thus obtained is brought under nitrogen into smaller preferably glass containers, such as bottles, which are suitable for the contemplated application.

## Claims

1. Antibiotic active aqueous composition especially for use by injection in the veterinary science, containing as the active antibiotic a complex of oxytetracycline and magnesium, polyvinylpyrrolidone as stabilisator and such a percentage of ethanolamine that the pH of the composition has a value of 7.0-9.5, characterized in that the composition contains as a solvent for the antibiotic active complex, glycofurol.

2. Composition according to claim 1, characterized in that the composition contains as an anti-oxydant a low percentage of sodiumforma ldehydesulfoxylate.

3. Composition according to claim 1 or 2, characterized in that monoethanolamine is used as the substance determining the pH.

4. Composition according to claim 1, 2 or 3, characterized in that in the antibiotic active complex the ratio by weight of the oxytetracycline/magnesium lies between 2 and 15.

5. Composition according to claim 1, 2, 3 or 4, characterized in that the following composition is fulfilled:
| | | |
|---|---|---|
| Oxytetracycline | 10-30 | % by weight |
| Magnesium | 1-15 | % by weight |
| sodiumformaldehydesulfoxylate | 0,2-1,0 | % by weight |
| Polyvinylpyrrolidone | 2-15 | % by weight |
| Glycofurol | 10-65 | % by weight |
| Monoethanolamine | 1-8 | % by weight |
| Water for injection q.s.p. | 100 | % by weight |
| the pH being between 7.0 - 9.5 | | |

6. Composition according to one or more of the claims 1-5, characterized in that the oxytetracycline is used in the form of oxytetracyclinedihydrate and/or oxytetracycline-hcl as a starting substance.
